# EUROPÄISCHE PATENTSCHRIFT

(11) **EP 1 045 014 B1**
(45) Veröffentlichungstag und Bekanntmachung des Hinweises auf die Patenterteilung: **03.08.2005**
(21) Anmeldenummer: 00107050.7
(22) Anmeldetag: 03.04.2000
(51) Int. Cl.: C09D 17/00, C09D 5/36, C09D 11/02, C09C 1/00, C08K 7/00, C09B 67/00

(54) **Pigmentmischung**
Pigment mixture
Mélange de pigments

(30) Priorität: 16.04.1999 DE 19917388
(43) Veröffentlichungstag der Anmeldung: 18.10.2000
(60) Teilanmeldung: 05010467.8
(73) Patentinhaber: MERCK PATENT GmbH, 64293 Darmstadt (DE)
(72) Erfinder: Anselmann, Ralf, Dr., 67305 Ramsen (DE); Pfaff, Gerhard, Dr., 64839 Münster (DE); Schoen, Sabine, Dr., 64287 Darmstadt (DE)

(56) Entgegenhaltungen:
- EP-A- 0 803 552
- DE-A- 19 618 566
- DE-A- 19 618 569
- US-A- 5 441 564

## Beschreibung

Die vorliegende Erfindung betrifft Pigmentmischungen gemäß Anspruch 1, sowie deren Verwendung in Lacken, Farben, Druckfarben, Kunststoffen, Pulverlacken, keramischen Glasuren, Kunststoffolien und kosmetischen Formulierungen.

Deckvermögen und Glanz sind bei plättchenförmigen Pigmenten oftmals nur schwer gleichzeitig in befriedigendem Ausmaß zu realisieren. So zeichnen sich etwa mit einer oder mehreren dünnen Metalloxidschichten belegte Glimmerplättchen oder SiO₂-Plättchen durch Interferenzfarben und einen hohen Glanz, gleichzeitig aber auch wegen des durchsichtigen Substrats durch eine hohe Transparenz und damit ein vergleichsweises geringes Deckvermögen, aus.

Aus der DE-A-42 40 511 ist eine Pigmentmischung bekannt, die sich aus einem Interferenzpigment und einem plättchenförmigen Farbpigment zusammensetzt. Das Interferenzpigment sind mit Metalloxiden beschichtete Glimmer- oder SiO₂-Plättchen und das Farbpigment können farbige SiO₂-Plättchen sein. Dieses Pigmentgemisch wird in Lacke, Druckfarben, Kunststoffe oder Kosmetika, eingearbeitet.

Aufgabe der vorliegenden Erfindung ist es eine Pigmentmischung bereitzustellen, die sich durch ein vergleichsweise hohes Deckvermögen auszeichnet und sich gut in das jeweilige Anwendungssystem einarbeiten läßt und bei der gleichzeitig eine Trennung Pigment/Farbmittel im System weitgehend ausgeschlossen ist.

Überraschenderweise wurde nun eine Pigmentmischung gefunden, die keine der oben angegebenen Nachteile aufweist. Das erfindungsgemäße Pigmentgemisch besteht aus mindestens zwei Komponenten, wobei Komponente A Mehrschichtpigmente (Multischichtpigmente) auf Basis von Glimmer, SiO₂-Plättchen, Glasplättchen, Al₂O₃-Plättchen oder Polymerplättchen, und Komponente B plättchenförmige, Farbmittel sind.

Durch die Zumischung eines oder mehrerer Farbmittel zu den Mehrschichtpigmenten (Multischichtpigmenten) kann den Anwendungssystemen ein Farbflop (stark winkelabhängiger Farbeindruck) verliehen werden, der Farbeffekt wird verstärkt und neuartige Farbeffekte werden erzielt.

Gegenstand der Erfindung ist somit ein Pigmentgemisch gemäß Anspruch 1.

Gegenstand der Erfindung sind ebenfalls die Formulierungen, wie z. B. Farben, Lacke, Druckfarben, Kunststoffe, Pulverlacke, keramische Glasuren, Kunststoffolien und kosmetische Zubereitungen, die das erfindungsgemäße Pigmentgemisch enthalten.

Das Verhältnis von Komponente A zu Komponente B ist 1 : 10 bis 10 : 1.

Die beispielsweise aus den deutschen Offenlegungsschriften DE 196 18 563, DE 196 18 566, DE 196 18 569, DE 197 07 805, DE 197 07 806, DE 197 46 067 bekannten Mehrschichtpigmente basieren auf einer plättchenförmigen, transparenten, farbigen oder farblosen Matrix, bestehend aus Glimmer (synthetisch oder natürlich), SiO₂-Plättchen, Glasplättchen, Al₂O₃-Plättchen, Polymerplättchen, und besitzen in der Regel eine Dicke zwischen 0,3 und 5 µm, insbesondere zwischen 0,4 und 2,0 µm. Die Ausdehnung in den beiden anderen Dimensionen beträgt üblicherweise zwischen 1 und 250 µm, vorzugsweise zwischen 2 und 100 µm, und insbesondere zwischen 5 und 40 µm. Die Mehrschichtpigmente bestehen aus der Matrix (Substrat) beschichtet mit Metalloxiden (mindestens 2). Die Beschichtung der Substratplättchen Glimmer, SiO₂-Plättchen, Glasplättchen, Al₂O₃-Plättchen mit mehreren Schichten erfolgt so, daß ein Schichtaufbau bestehend aus alternierenden hoch- und niedrigbrechenden Schichten entsteht. Vorzugsweise enthalten die Mehrschichtpigmente 2, 3, 4, 5, 6 oder 7 Schichten, insbesondere 3, 4 oder 5 Schichten. Geeignete hochbrechende Metalloxide sind beispielsweise Titandioxid, Zirkonoxid, Zinkoxid, Eisenoxide, Eisen-Titan-Oxide (Eisentitanate) und/oder Chromoxid, insbesondere TiO₂ und/oder Fe₂O₃. Als niedrigbrechende Metalloxide kommen SiO₂ und Al₂O₃ zum Einsatz. Es kann hierfür jedoch auch MgF₂ oder ein organisches Polymer (z.B. Acrylat) eingesetzt werden. Die Beschichtung der Substratplättchen kann z.B. erfolgen wie in der WO 93/08237 (naßchemische Beschichtung) oder DE-OS-196 14 637 (CVD-Verfahren) beschrieben.

Bevorzugte Mehrschichtpigmente besitzen folgenden Aufbau:
Substrat + Fe₂O₃-Schicht + SiO₂-Schicht + Fe₂O₃-Schicht
Substrat + Fe₂O₃-Schicht + SiO₂-Schicht + TiO₂-Schicht
Substrat + TiO₂-Schicht + SiO₂-Schicht + Fe₂O₃-Schicht
Substrat + TiO₂-Schicht + SiO₂-Schicht + TiO₂/Fe₂O₃-Schicht
Substrat + TiO₂/Fe₂O₃-Schicht + SiO₂-Schicht + TiO₂/Fe₂O₃-Schicht
Substrat + TiO₂-Schicht + SiO₂-Schicht + Cr₂O₃-Schicht

Anstelle der äußeren Metalloxidschicht kann auch eine semitransparente Schicht eines Metalls verwendet werden. Geeignete Metalle dafür sind beispielsweise Cr, Ti, Mo, W, Al, Cu, Ag, Au oder Ni.

Zur Erzielung spezieller Farbeffekte können in die hoch- bzw. niedrigbrechenden Schichten zusätzlich noch feinteilige Partikel im Nanometergrößenbereich eingebracht werden. Als geeignet dafür erweisen sich beispielsweise feinteiliges TiO₂ oder feinteiliger Kohlenstoff (Ruß) mit Teilchengrößen im Bereich von 10-250 nm. Durch die lichtstreuenden Eigenschaften derartiger Partikel kann gezielt auf Glanz und Deckvermögen Einfluß genommen werden.

Die Mehrschichtpigmente können auch zur Verbesserung der Licht-, Wetter- und chemischen Stabilität oder zur Erhöhung der Kompatibilität in unterschiedliche Medien noch mit einer Schutzschicht versehen sein. Als Nachbeschichtungen bzw. Nachbehandlungen kommen beispielsweise die in den DE 22 15 191, DE 31 51 354, DE 32 35 017 oder DE 33 34 598 beschriebenen Verfahren in Frage. Die zusätzlich aufgebrachten Stoffe machen nur etwa 0,1 bis 5 Gew.-%, vorzugsweise 0,5 bis 3,0 Gew.-%, des Mehrschichtpigments aus.

Als Komponente B für die erfindungsgemäße Pigmentmischung sind alle dem Fachmann bekannten plättchenförmigen, Farbmittel geeignet, die eine Partikelgröße von 0,001 bis 10 µm, vorzugsweise 0,01 bis 1 µm, aufweisen.

Pigmentgemische enthalten neben Komponente A als Farbmittel (Komponente B) ein Perlglanzpigment. Als Perlglanzpigmente werden Pigmente auf der Basis plättchenförmiger, transparenter oder semitransparenter Substrate aus z.B. Schichtsilikaten, wie etwa Glimmer, synthetischem Glimmer, oder Glas verwendet, die mit farbigen oder farblosen Metalloxiden wie z.B. TiO₂, Titansuboxide, Titanoxinitride, Fe₂O₃, Fe₃O₄, SnO₂, Cr₂O₃, ZnO, CuO, NiO und anderen Metalloxiden allein oder in Mischung in einer einheitlichen Schicht beschichtet sind. Perlglanzpigmente sind z.B. aus den deutschen Patenten und Patentanmeldungen 14 67 468, 19 59 998, 20 09 566, 22 14 454, 22 15 191, 22 44 298, 23 13 331, 25 22 572, 31 37 808, 31 37 809, 31 51 343, 31 51 354, 31 51 355, 32 11 602, 32 35 017 und P 38 42 330 bekannt und im Handel erhältlich, z.B. unter der Marke Iriodin® der Firma Merck KGaA, Darmstadt, Deutschland. Besonders bevorzugte Pigmentpräparationen enthalten TiO₂/Glimmer-, Fe₂O₃/Glimmer- und/oder TiO₂/Fe₂O₃-Glimmerpigmente.

Weiterhin bevorzugt sind mit TiO₂ und/oder Fe₂O₃ beschichtete SiO₂- oder Al₂O₃-Plättchen. Die Beschichtung der SiO₂-Plättchen mit ein oder mehreren Metalloxiden kann z.B. erfolgen wie in der WO 93/08237 (naßchemische Beschichtung) oder DE-OS 196 14 637 (CVD-Verfahren) beschrieben.

Als plättchenförmige Farbmittel kommen Perlglanzpigmente auf Basis von Glimmer, SiO₂-Plättchen, Al₂O₃-Plättchen oder Glas-Plättchen, die nur mit einer Metalloxidschicht umhüllt sind, in Frage.

Die erfindungsgemäße Pigmentmischung ist einfach und leicht zu handhaben. Die Pigmentmischung kann durch einfaches Einrühren in das Anwendungssystem eingearbeitet werden. Ein aufwendiges Mahlen und Dispergieren der Pigmente ist nicht erforderlich.

Die erfindungsgemäße Pigmentmischung kann zur Pigmentierung von Lacken, Druckfarben, Kunststoffen, Agrarfolien, Saatgutbeschichtung, Lebensmitteleinfärbungen, Knopfpasten, Arzneimittelüberzügen oder kosmetischen Formulierungen, wie Lippenstifte, Nagellacke, Preßpuder, Shampoos, lose Puder und Gele, verwendet werden. Die Konzentration der Pigmentmischung im zu pigmentierenden Anwendungssystem liegt in der Regel zwischen 0,1 und 70 Gew.%, vorzugsweise zwischen 0.1 und 50 Gew.% und insbesondere zwischen 1,0 und 10 Gew.%, bezogen auf den Gesamtfestkörpergehalt des Systems. Sie ist in der Regel abhängig vom konkreten Anwendungsfall.

Kunststoffe enthaltend das erfindungsgemäße Pigmentgemisch in Mengen von 0,01 bis 50 Gew. %, insbesondere 0,1 bis 7 Gew.%, zeichnen sich häufig durch einen besonderen Farbflop-Effekt, aber auch durch einen Sparkle-Effekt aus.

Im Lackbereich, insbesondere im Automobillack, wird das Pigmentgemisch, auch für 3-Schichtaufbauten in Mengen von 0,1-10 Gew.%, vorzugsweise 1 bis 3 Gew.%, eingesetzt. Das Mischungsverhältnis der Mehrschichtpigmente mit Komponente B hängt vom gewünschten Effekt ab. Die Mehrschichtpigment werden mit Komponente B im Verhältnis von 10 : 1, bis 1 : 10, eingesetzt.

Im Lack hat die erfindungsgemäße Pigmentmischung den Vorteil, daß der angestrebte Farbflop-Effekt durch eine einschichtige Lackierung (Einschichtsystem bzw. Base coat im 2-Schichtaufbau) erzielt wird. Dieser Farbflop ist auch bei diffusem Licht sehr deutlich ausgeprägt. Im Vergleich mit Lackierungen, die ein Interferenzpigment auf Basis von Glimmer enthalten statt der Mehrschichtpigmente, zeigen Lackierungen mit der erfindungsgemäßen Pigmentmischung eine deutlichere Tiefenwirkung und einen Glitzereffekt.

Das erfindungsgemäße Pigmentgemisch kann auch vorteilhaft in der dekorativen und pflegenden Kosmetik eingesetzt werden. Die Einsatzkonzentration und das Mischungsverhältnis von Mehrschichtpigmenten mit Komponente B, sind abhängig vom Anwendungsmedium und dem Effekt, der erzielt werden soll. Die Mischung aus Mehrschichtpigmenten mit anderen Pigmenten oder Farbstoffen erfoglt im Verhältnis 1 : 10 bis 10 : 1. Die Einsatzkonzentration reicht von 0,01 Gew.% im Shampoo bis zu 70 Gew. % im Preßpuder. Bei einer Mischung von Mehrschichtpigmenten mit sphärischen Füllstoffen, z. B. SiO₂, kann die Konzentration bei 0,01-70 Gew.% in der Formulierung liegen. Die kosmetischen Produkte, wie z.B. Nagellacke, Lippenstifte, Preßpuder, Shampoos, lose Puder und Gele, zeichnen sich durch besonders interessante Glanz- und/oder Farbeffekte aus, die je nach Aufbau des Mehrschichtpigments stark winkelabhängig sein können. Der Glitzereffekt in Nagellack kann gegenüber herkömmlichen Nagellacken mit Hilfe der erfindungsgemäßen Pigmentgemische deutlich gesteigert werden. Weiterhin kann das erfindungsgemäße Pigmentgemisch in Badezusätzen, Zahnpasten und zur Veredlung von Lebensmitteln, z. B. Masse-Einfärbung oder als Überzug, eingesetzt werden.

Bei der Pigmentierung von Bindemittelsystemen z. B. für Farben und Druckfarben für den Tiefdruck, Offsetdruck oder Siebdruck, oder als Vorprodukt für Druckfarben, z.B. in Form von hochpigmentierten Pasten, Granulaten, Pellets, etc., haben sich insbesondere Pigmentgemische bestehend aus Mehrschichtpigmenten mit sphärischen Farbmitteln, wie z.B. TiO₂, Ruß, Chromoxid, Eisenoxid sowie organische "Farbpigmente" als besonders geeignet erwiesen. Das Pigmentgemisch gemäß Anspruch 1 wird in der Regel in die Druckfarbe in Mengen von 2-35 Gew.%, vorzugsweise 5-25 Gew.%, und insbesondere 8-20 Gew.% eingearbeitet. Offsetdruckfarben können das Pigmentgemisch bis zu 40 Gew.% und mehr enthalten. Die Vorprodukte für die Druckfarben, z.B. in Granulatform, als Pellets, Briketts, etc., enthalten neben dem Bindemittel und Additiven bis zu 95 Gew.% des erfindungsgemäßen Pigmentgemisches. Das Mischungsverhältnis von Komponente A zu Komponente B liegt im Bereich von 1 : 10 bis 10 : 1. Die Druckfarben enthaltend das erfindungsgemäße Pigmentgemisch zeigen reinere Farbtöne und sind aufgrund der guten Werte für die Viskosität besser verdruckbar.

Gegenstand der Erfindung sind somit auch Formulierungen enthaltend das erfindungsgemäße Pigmentgemisch.

Die nachfolgenden Beispiele sollen die Erfindung erläutern, ohne sie jedoch zu begrenzen.

### Beispiele

### Vergleichs Beispiel 1: - Druckfarbe

Das Pigment wurde bei 600 Upm in das lösemittelhaltige Bindemittel eingerührt und die Druckfarben wurden anschließend auf Schwarz-Weiß-Karten aufgerakelt.

| Farbe Nr. 1: | |
|---|---|
| 88,0 g | Gebr. Schmidt 95 MB 011 TW |
| 10,0 g | Mehrschichtpigment bestehend aus Glimmerplättchen der Teilchengröße 5-40 µm, beidseitig mit 3 Schichten in der Folge Fe₂O₃, SiO₂, Fe₂O₃ |
| 2,0 g | Gebr. Schmidt 95 MB 022-TW (Grün) |

| Farbe Nr. 2: Vergleich | |
|---|---|
| 88,0 g | Gebr. Schmidt 95 MB 011 TW |
| 10,0 g | mit Fe₂O₃ beschichteter Glimmer der Teilchengröße 10 bis 60 µm (Fa. Merck KGaA) |
| 2,0 g | Gebr. Schmidt 95 MB 022-TW (Grün) |

Die Farbkarte mit der Farbe Nr. 1 zeigt visuell einen deutlich besseren Farbflop als die Farbkarten mit der Vergleichsfarbe Nr. 2.

### Veigleichs Beispiel 2: - Autolack

| | |
|---|---|
| 2,0 g | SiO₂-Plättchen der Teilchengröße 5-40µm, beidseitig beschichtet mit 3 Schichten in der Folge Fe₂O₃, SiO₂, Fe₂O₃ |
| 1,5 g | Heliogenblau L 6930 |
| 0,2 g | Hostapermgrün 8G |
| 0,05 g | Farbruß FW 200 |
| 66,6 g | Basislack (A4) MP-System (FK = 19 %) |
| 29,65 g | Verdünnermischung |

Es wird ein Farbton mit einem stark ausgeprägten Blau-Grün-Umschlag und goldenen Highlights erreicht, der sogar bis Richtung Dunkelviolett changieren kann.

### Vergleichs Beispiel 3: - Kunststoff

Den Kunststoffgranulaten Polypropylen PP Stamylan PPH10 (Fa. DSM) bzw. Polystyrol 143E (Fa. BASF) werden jeweils
a) 1 % SiO₂-Plättchen der Teilchengröße 5-40 µm, beidseitig beschichtet mit 3 Schichten der Folge Fe₂O₃, SiO₂, Fe₂O₃
b) eine Mischung aus
   1 % SiO₂-Plättchen der Teilchengröße 5 - 40 µm, beidseitig beschichtet mit 3 Schichten in der Folge Fe₂O₃, SiO₂, Fe₂O₃ und 0,1 % PV-Echtblau B2G01 (Pigment Blue 15,3 der Fa. Clariant)
c) 1 % mit Fe₂O₃ beschichteter Glimmer der Teilchengröße 10 - 60 µm (Merck KGaA)
d) eine Mischung aus
   1 % mit Fe₂O₃ beschichteter Glimmer der Teilchengröße 10 - 60 µm (Merck KGaA) und 0,1 % PV-Echtblau B2G01 (Pigment Blue 15,3 der Fa. Clariant)
zugegeben.

Das pigmentierte Granulat wird anschließend auf einer Spritzmaschine zu Stufenplättchen verarbeitet.

Bei Verwendung des Pigments unter a) und bei der Mischung unter b) wird ein stark winkelabhängiger Farbeindruck des Kunststoffs erreicht. Dieser ist im Fall von b) noch deutlich stärker variierbar und kann z. B. für einen kräftigen Blau-Grün-Umschlag verwendet werden.

### Vergleichs Beispiel 4: - Lidschatten

| Phase I | |
|---|---|
| 5,00 % | Silica |
| 25,00 % | SiO₂-Plättchen der Teilchengröße 5-40 µm beschichtet mit 3 |
| | Schichten in der Folge Fe₂O₃, SiO₂, Fe₂O₃ |
| 5,00 % | C.I. Pigment Green 18 (CI77289) |
| 47,42 % | Talc |
| 7,18 % | Solanum Tuberosum (Potato Starch) |
| 2,40 % | Magnesium Stearate |

| Phase II | |
|---|---|
| 6,96 % | Isopropyl Stearate |
| 0,40 % | Cetyl Palmitate |
| 0,40 % | Petrolatum |
| 0,08 % | Konservierung |

Die Bestandteile der Phase I werden zusammengegeben und vorgemischt. Anschließend wird die Pudermischung unter Rühren tropfenweise mit der geschmolzenen Phase II versetzt. Die Puder werden bei 40-50 bar gepreßt.

Der erhaltene Lidschatten zeichnet sich durch einen intensiven Glanz und einen stark changierenden Farbeindruck je nach Blickwinkel aus.

### Vergleichs Beispiel 5: - Lippenstift

| Phase I | |
|---|---|
| 8,25 % | Cera alba |
| 4,95 % | Ceresin, Copernica Cerifera |
| 3,30% | Lanolin Oil |
| 5,28 % | Isopropyl Myristate |
| 1,98 % | Mineral Oil |
| 0,03 % | Tocopherol, Ascorbyl Palmitate, Ascorbic Acid, Citric Acid, |
| | PEG-8 |
| 0,06 % | Konservierung |
| 0,50 % | Aroma |
| 1,00 % | Lithalrubin BK, C.I. Pigment Red 57:1 (CI 15850) |
| ad 100,00 % | Ricinus Communis (20 % in Castor Oil) |

| Phase II | |
|---|---|
| 2,00 % | Silica |
| 15,00 % | SiO₂-Plättchen der Teilchengröße 5-40 µm beschichtet mit 3 Schichten in der Folge Fe₂O₃, SiO₂, Fe₂O₃ |

Die Bestandteile der Phase I werden auf 75 °C erhitzt und aufgeschmolzen. Die Pigmente der Phase II werden zugegeben und alles gut durchgerührt. Die Lippenstiftmasse wird dann in der auf 65 °C temperierten Gießapparatur 15 Min. gerührt. Die homogene Schmelze wird in die auf 65 °C vorgewärmten Gießformen gegossen. Anschließend kühlt man die Formen ab und entfernt die Gießlinge kalt.

Der Lippenstift zeichnet sich durch einen intensiven Glanz und einen stark changierenden Farbeindruck je nach Blickwinkel aus.

### Vergleichs Bespiel 6: - Duschgel

| Phase A | |
|---|---|
| 0,10 % | Glimmerpigment der Teilchengröße 10-60 µm, beidseitig beschichtet mit TiO₂, SiO₂, TiO₂ (1) |
| 0,75 % | Xanthan Gum (2) |
| 62,10% | Wasser |

| Phase B | |
|---|---|
| 20,00 % | Decyl Glucoside (3) |
| 6,65 % | Texapon ASV (Sodium Laureth Sulfate, Magnesium Laureth Sulfate, Sodium Laureth 8-Sulfate, Magnesium Laureth 8-Sulfate, Sodium Oleth Sulfate, Magnesium Oleth Sulfate (3) |
| 0,20 % | Bronidox L (Propylene Glycol, 5-Bromo-5-nitro-1,3-dioxane)(3) |
| 0,05 % | Parfüm (Parfümöl Everest 79658 SB) (4) |

| Phase C | |
|---|---|
| 0,15 % | Citric Acid (1) |
| 0,10 % | Sicovit Grün Z 6120 (1 %ig in Wasser) [CI 19140/42051] (5) |
| 10,00 % | Wasser |

### Bezugsquellen:

(1) Merck KGaA
(2) Monsanto
(3) Henkel KGaA
(4) Haarmann & Reimer
(5) BASF

Für Phase A wird das Pigment in Wasser eingerührt. Xanthan Gum wird unter Rühren langsam in die Suspension eingestreut und solange gerührt bis es gelöst ist. Die Phasen B und C werden nacheinander hinzugefügt und anschließend wird solange gerührt bis alles homogen verteilt ist.

Das Duschgel zeichnet sich durch intensive Interferenz-Glitzereffekte aus.

### Beispiel 1: - Eyeliner Gel

| Phase A | |
|---|---|
| 15,00 % | Glimmerpigment der Teilchengröße 10-60 µm, beidseitig beschichtet mit TiO₂, SiO₂, TiO₂ (1) |
| 5,0 % | Glimmerpigment der Teilchengröße 10-60 µm beschichtet mit Fe₃O₄ (1) |
| 2,00 % | Ronasphere® (Silica) (1) |
| 0,40 % | Carbopol ETD 2001 (Carbomer) (2) |
| q.s. | Citric acid (1) |
| 60,00 % | Wasser |

| Phase B | |
|---|---|
| 4,0 % | Glycerin (1) |
| 0,90 % | Triethanolamine (1) |
| 2,00 % | Luviskol VA 64 Pulver (PVPNA Copolymer) (3) |
| 0,20 % | Germaben II (Propylene Glycol, Diazolidinyl Urea, Methylparaben, Propylparaben) (4) |
| ad 100,00 % | Wasser |

### Bezugsquellen:

(1) Merck KGaA
(2) BF Goodrich
(3) BASF
(4) ISP Europe

Die Pigmente und Ronasphere® werden im Wasser der Phase A dispergiert. Mit einigen Tropfen Zitronensäurelösung wird der pH auf 3,8 eingestellt um die Viskosität zu vermindern. Carbopol wird unter Rühren eingestreut. Nach vollständiger Lösung wird die vorgelöste Phase B langsam eingerührt.

Das erhaltene Produkt zeichnet sich durch hohen Glanz und intensive Interferenzfarbe aus.

## Patentansprüche

**1.** Pigmentgemisch bestehend aus mindestens zwei Komponenten A und B, die im Verhältnis 10 : 1 bis 1 : 10 gemischt sind, wobei Komponente A Mehrschichtpigmente (Multischichtpigmente) auf Basis von Glimmer, SiO₂-Plättchen, Glas-Plättchen, Al₂O₃-Plättchen oder Polymerplättchen beschichtet mit altemierenden Schichten aus hoch- und niedrigbrechenden Metalloxiden und Komponente B Perlglanzpigmente auf Basis von Glimmer, SiO₂-Plättchen, Al₂O₃-Plättchen oder Glasplättchen, die mit einer Metalloxidschicht umhüllt sind, sind.

**2.** Pigmentgemisch nach Anspruch 1, **dadurch gekennzeichnet, daß** das Mehrschichtpigment als hochbrechende Schicht ein oder mehrere TiO₂-, Fe₂O₃-, ZnO- und/oder ZrO₂-Schichten enthält.

**3.** Pigmentgemisch nach Anspruch 1, **dadurch gekennzeichnet, daß** das Mehrschichtpigment als niedrigbrechende Schicht ein oder mehrere SiO₂- und/oder Al₂O₃-Schichten enthält.

**4.** Pigmentgemisch nach einem der Ansprüche 1 bis 3 **dadurch gekennzeichnet, daß** Komponente A ein Mehrschichtpigment enthält, das eine Fe₂O₃-, SiO₂-, Fe₂O₃-Beschichtung, eine TiO₂/Fe₂O₃-, SiO₂-, TiO₂/Fe₂O₃-Beschichtung oder eine TiO₂-, SiO₂-, TiO₂-Beschichtung aufweist.

**6.** Verwendung des Pigmentgemisches nach Anspruch 1 in Farben, Lacken, insbesondere Automobillacken, Druckfarben, Kunststoffen, Pulverlacken, zur Saatguteinfärbung, in kosmetischen Formulierungen und zur Veredelung von Lebensmitteln.

**7.** Formulierungen enthaltend ein Pigmentgemisch nach Anspruch 1.

## Claims

**1.** Pigment mixture consisting of at least two components A and B, which are mixed in the ratio 10 : 1 to 1 : 10, where component A comprises multilayered pigments based on mica, SiO₂ platelets, glass platelets, Al₂O₃ platelets or polymer platelets coated with alternating layers of high- and low-refracting metal oxides and component B comprises pearlescent pigments based on mica, SiO₂ platelets, Al₂O₃ platelets or glass platelets sheathed with a metal-oxide layer.

**2.** Pigment mixture according to Claim 1, **characterised in that** the multilayered pigments comprises, as high-refracting layer, one or more TiO₂, Fe₂O₃, ZnO and/or ZrO₂ layers.

**3.** Pigment mixture according to Claim 1, **characterised in that** the multilayered pigment comprises, as low-refracting layer, one or more SiO₂ and/or Al₂O₃ layers.

**4.** Pigment mixture according to one of Claims 1 to 3, **characterised in that** component A comprises a multilayered pigment which has an Fe₂O₃, SiO₂, Fe₂O₃ coating, a TiO₂/ Fe₂O₃, SiO₂, TiO₂/ Fe₂O₃ coating or a TiO₂, SiO₂, TiO₂ coating.

**6.** Use of the pigment mixture according to Claim 1 in coatings, paints, in particular automobile paints, printing inks, plastics, powder coatings, for colouring seed, in cosmetic formulations and for processing foods.

**7.** Formulations comprising a pigment mixture according to Claim 1.

## Revendications

**1.** Mélange de pigments constitué par au moins deux composants A et B, lesquels sont mélangés selon un rapport de 10 : 1 à 1 : 10, dans lequel le composant A est constitué par des pigments multicouches basés sur du mica, des plaquettes de SiO₂, des plaquettes de verre, des plaquettes d'Al₂O₃ ou des plaquettes de polymère qui sont revêtues par des couches alternées d'oxydes métalliques à réfractions haute et basse et le composant B est constitué par des pigments perlés basés sur du mica, des plaquettes de SiO₂, des plaquettes d'Al₂O₃ ou des plaquettes de verre qui sont enveloppées par une couche d'oxyde métallique.

**2.** Mélange de pigments selon la revendication 1, **caractérisé en ce que** les pigments multicouches comprennent, en tant que couche à réfraction haute, une ou plusieurs couches de TiO₂, Fe₂O₃, ZnO et/ou ZrO₂.

**3.** Mélange de pigments selon la revendication 1, **caractérisé en ce que** les pigments multicouches comprennent, en tant que couche à réfraction basse, une ou plusieurs couches de SiO₂ et/ou d'Al₂O₃.

**4.** Mélange de pigments selon l'une des revendications 1 à 3, **caractérisé en ce que** le composant A comprend un pigment multicouche qui présente un revêtement Fe₂O₃, SiO₂, Fe₂O₃, un revêtement TiO₂/Fe₂O₃, SiO₂, TiO₂/ Fe₂O₃ ou un revêtement TiO₂, SiO₂, TiO₂.

**6.** Utilisation du mélange de pigments selon la revendication 1 dans des revêtements, des peintures, en particulier des peintures d'automobile, des encres d'impression, des plastiques, des revêtements pulvérulents, pour colorer des graines, dans des formulations cosmétiques et pour le traitement d'aliments.

**7.** Formulations comprenant un mélange de pigments selon la revendication 1.
